# EUROPÄISCHE PATENTSCHRIFT

(11) **EP 1 438 014 B1**
(45) Veröffentlichungstag und Bekanntmachung des Hinweises auf die Patenterteilung: **28.05.2008**
(21) Anmeldenummer: 02777220.1
(22) Anmeldetag: 26.09.2002
(51) Int. Cl.: A61K 8/06, A61K 8/34, A61K 31/07, A61Q 19/08, A61P 17/16

(54) **O/W-EMULSIONEN MIT UNPOLAREN WIRKSTOFFEN**
OIL-IN-WATER EMULSION CONTAINING NON-POLAR ACTIVE PRINCIPLES
EMULSIONS HUILE DANS L'EAU CONTENANT DES PRINCIPES ACTIFS NON POLAIRES

(30) Priorität: 04.10.2001 DE 10148825
(43) Veröffentlichungstag der Anmeldung: 21.07.2004
(60) Teilanmeldung: 07019440.2
(73) Patentinhaber: Beiersdorf AG, 20253 Hamburg (DE)
(72) Erfinder: NIELSEN, Jens, 24558 Henstedt-Ulzburg (DE); RASCHKE, Thomas, 22529 Pinneberg (DE)
(86) Internationale Anmeldenummer: PCT/EP2002/010807
(87) Internationale Veröffentlichungsnummer: WO 2003/030859

(56) Entgegenhaltungen:
- EP-A2- 1 048 292
- WO-A1-98/14167
- WO-A1-99/53894
- DE-A1- 19 645 319

## Beschreibung

Die vorliegende Erfindung betrifft Hautpflegeprodukte auf Emulsionsbasis, die unpolare und/oder ungesättigte Wirkstoffe enthalten.

Als unpolare und/oder ungesättigte kosmetische und/oder dermatologische Wirkstoffe sind beispielsweise Retinoide, Ubichinone und Carotinoide bekannt. Diese zeichen sich unter anderem durch einen logP-Wert von mindestens 3,5 aus. Zahlreiche Vertreter dieser Gruppe von Wirkstoffen zeichnen sich durch eine besondere kosmetische Wirksamkeit aus, lassen sich aber oft schwer in kosmetische und/oder dermatologische Zubereitungen einarbeiten.

Retinoide sind Isoprenoide mit 20 Kohlenstoffatomen, die einen ungesättigten Cyclohexanring tragen, dessen Doppelbindung in Konjugation mit den Doppelbindungen des verzweitgkettigen Kohlenwasserstoffrestes stehen. Die namensgebende Substanz Retinol kommt in sehr hohen Anteilen im Fischöl vor, wird aber heute synthetisch hergestellt und in großen Mengen z.B. als Futtermittelzusatz verwendet.
Als Retinoide im Sinne der vorliegenden Schrift werden begrifflich auch alle kosmetisch und/oder pharmazeutisch unbedenklichen Retinoide, einschließlich des Retinols und seiner Ester, des Retinals sowie der Retinsäure und deren Ester einbezogen.

Retinol ist durch folgende Struktur gekennzeichnet: 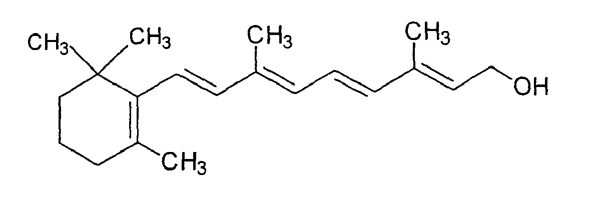 Retinol (auch: Axerophthol; [3,7-Dimethyl-9-(2,6,6-trimethyl-1-cyclohexenyl)-2,4,6,8-nonatetraen-1-ol) ist synonym mit Vitamin A₁, wird auch analog den Derivaten Retin-1-carbonsäure (Vitamin-A-Säure, Retinsäure, Tretinoin) und deren Estern bzw. dem Retin-1-al (Vitamin-A-aldehyd) gelegentlich Vitamin-A-Alkohol genannt.

Bedeutend sind auch Retinolester, die durch folgende Struktur gekennzeichnet sind: 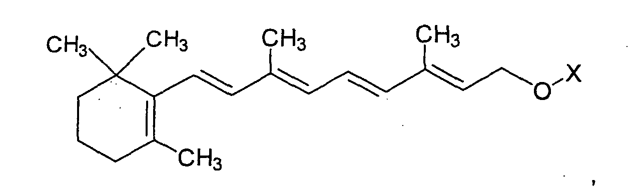 wobei X bevorzugt einen verzweigten oder unverzweigten Alkanoyl- oder Alkenoylrest darstellt. Retinal ist durch die Struktur 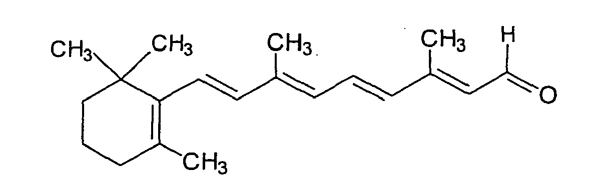 gekennzeichnet. Retinal [Vitamin A1-Aldehyd, 3,7-Dimethyl-9-(2,6,6-trimethyl-1-cyclohexenyl)- 2,4,6,8- nonatetraenal] ist in seiner all-trans-Form am stabilsten. Das früher Retinen genannte Retinal bildet, an Opsine gebunden, die Sehpigmente Rhodopsin und lodopsin sowie das andere Funktionen wahrnehmende Bakteriorhodopsin. Retinal entsteht durch oxidative Spaltung des Carotins.

Retinsäure [Vitamin-A-Säure, all-trans-3,7-Dimethyl-9-(2,6,6-trimethyl- 1- cyclohexenyl)-2,4,6,8- nonatetraensäure] ist durch die Struktur 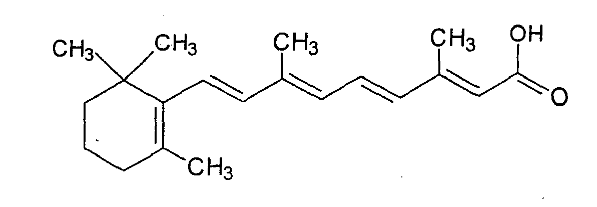 gekennzeichnet. Sie ist durch Hemmung der Talgproduktion wirksam bei besonders schweren Akne-Fällen, wirkt allerdings teratogen. Gleichwohl kann in bestimmten medizinisch indizierten Fällen die Verwendung der Retinsäure bzw. ihrer Ester vorteilhaft sein und ist insofern in solchen Fällen als "unbedenklich" anzusehen.

Retinoide sind oxidationempfindliche Wirkstoffe. Um sie stabil in Zubereitungen zu formulieren, wurden verschiedene Wege vorgeschlagen, beispielsweise molekulare Verkapselung. So lehrt die DE 19943678 Verwendung von in Cyclodextrinen verkapselten Retinoiden in bestimmten Wirkstoffkombinationen. Eine weitere Methode ist die Verwendung luftundurchlässiger Packmittel wie Tuben aus Aluminium.

Eine weitere wichtige Klasse von lipophilen, konjugiert ungesättigten Wirkstoffen sind die Carotinoide. "Carotinoid" ist die von Carotin abgeleitete Bezeichnung für Carotine (reine Kohlenwasserstoffe) und Xanthophylle (Sauerstoff-haltige Carotine), deren Grundgerüst aus acht Isopren-Einheiten (Tetraterpene) besteht. Man kann sich die Carotinoide aus zwei C₂₀-Isoprenoiden derart zusammengesetzt denken, daß die beiden mittleren Methyl-Gruppen in 1,6-Stellung zueinander stehen (Kopf-Kopf-Verknüpfung); die beiden Mol.-Hälften gehorchen jeweils der Isopren-Regel.

Der bekannteste Vertreter der Carotinoide ist das β-Carotin , welches sich durch die folgende Strukturformel auszeichnet: 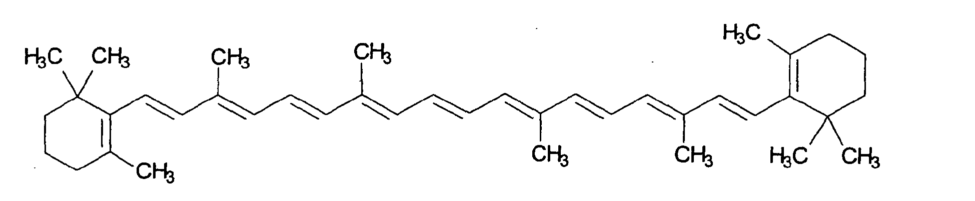 und einen log P-Wert von etwa 15 hat.

Ubichinone zeichnen sich durch die Strukturformel 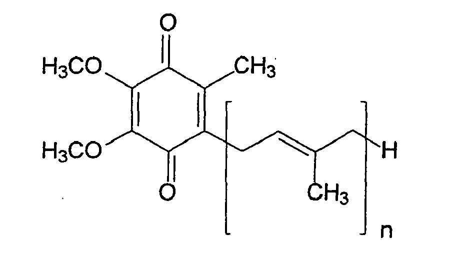 aus und stellen die am weitesten verbreiteten u. damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen u. Hefen mit n=6. Bei den meisten Säugetieren einschließlich des Menschen überwiegt Q-10.

Coenzym Q-10 beispielsweise ist durch folgende Strukturformel gekennzeichnet: 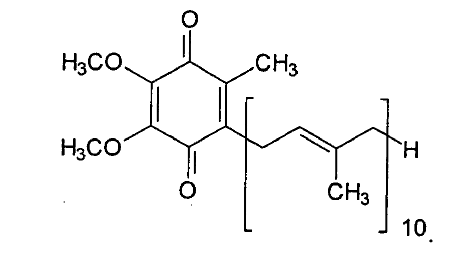 Ubichinone dienen den Organismen als Elektronenüberträger in der Atmungskette. Sie befinden sich in den Mitochondrien, wo sie die cyclische Oxidation und Reduktion der Substrate des Citronensäure-Cyclus ermöglichen.

Plastochinone weisen die allgemeine Strukturformel 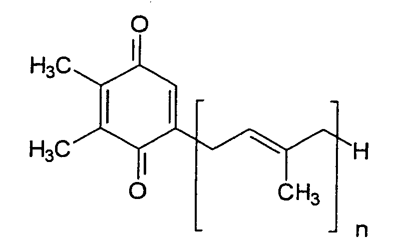 auf. Sie können aus Chloroplasten isoliert werden und spielen als Redoxsubstrate in der Photosynthese beim cyclischen und nichtcyclischen Elektronentransport eine Rolle, wobei sie reversibel in die entsprechenden Hydrochinone (Plastochinol) übergehen. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Kosmetische Zubereitungen mit Coenzym Q-10 aus der DE-A-33 09 850 sind bekannt, die zur Behandlung von Hautkrankheiten, zur Prophylaxe von dystrophischen und dysmetabolischen Zuständen der Haut und zur Anwendung bei chemischen und physikalischen Respirationsschäden oder bei verzögerter Respiration verbunden mit Alter und Abnutzung geeignet sind.

In der japanischen Offenlegungsschrift 58,180,410 ist die Eignung von Coenzym Q-10 für Kosmetika beschrieben. Es soll den Hautzellmetabolismus aktivieren und die Oxidation unterdrücken. Coenzym Q10 hat im Resultat eine wichtige Funktion bei der Prävention von Hautschäden durch UV-Strahlen und der Prävention von Hautalterung.

Mischungen aus
(a) einer oder mehreren grenzflächenaktiven Substanzen A, gewählt aus der Gruppe der Glucosederivate, welche sich durch die Strukturformel 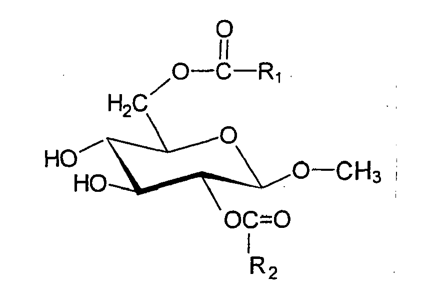 auszeichnen, wobei R einen verzweigten oder unverzweigten Alkylrest mit 1 bis 24 Kohlenstoffatomen darstellt, wobei R₁ und R₂ unabhängibg voneinander entweder ein Wasserstoffatom oder einen verzweigten oder unverzweigten Alkylrest mit 1 bis 24 Kohlenstoffatomen darstellen, enthalten,
(b) einer oder mehreren grenzflächenaktiven Substanzen B, gewählt aus der Gruppe der Substanzen der allgemeinen Strukturformel 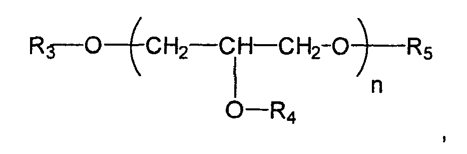 wobei R₃, R₄ und R₅ voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt,
sind als Emulgatoren an sich bekannt.

Bewährt haben sich etwa äquimolare Mischungen aus Methylglucosedistearat (Formel wie nachstehend) 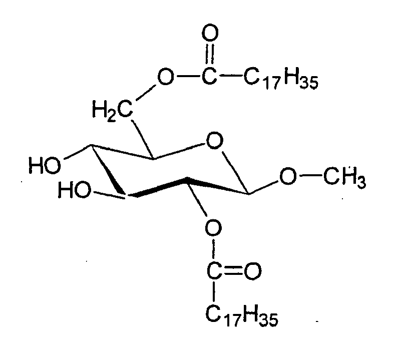 und Triglyceryldicarboxylaten mit der generischen Formel 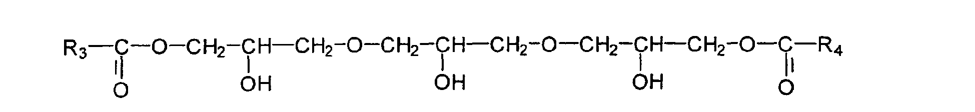 bei welcher R₃ und R₄ unabhängig voneinander gewählt werden aus der Gruppe, welche umfaßt: H, verzweigte bzw. unverzweigte, gesättigte bzw. ungesättigte Fettsäurereste mit 14 bis 20 Kohlenstoffatomen.

Besonders bewährt haben sich etwa äquimolare Miaschungen aus Methylglucosedistearat und Triglyceryldicarboxylaten, bei denen die Reste R₃ und R₄ vorzugsweise beide einen Stearatrest bezeichnen. Solche Emulgatorkombinationen sind als "Polyglyceryl(3)-Methylglucosedistearat" (PGMS) unter der Warenbezeichnung Tego Care® 450 von der Gesellschaft Th. Goldschmidt KG erhältlich.

Die Schrift WO 98/52536 beschreibt Zubereitungen zur Hautpflege, die Retinoide und bestimmte Konservierungsmittel enthalten.

Die Schriften WO 96/7396 und WO 98/25587 beschreiben näher beschriebene Zubereitungen zur Hautpflege, die Retinoide und ein bestimmtes Stabilisatorsystem enthalten.

Die Schrift EP1023892 beschreibt die Stabilisierung von Retinoiden mit Hilfe von Zitronensäure und bestimmten Fettsäureglyceriden.

Die Schrift WO 95/26180 beschreibt topische Zubereitungen mit einem Gehalt an Vitamin-A-Säure und wahlweise Ubichinonen und/oder Plastochinonen, wobei alle Zubereitungen auch in Form iherer Derivate vorliegen können. über eine Stabilisierung der lipophilen Wirkstoffe wird aber nichts offenbart,

Die Schrift WO 98/25587 beschreibt ein näher bestimmtes Verfahren zur topischen Anwendung von Retinoiden mit lipophilen Antioxidantien.

Die Schrift EP 839520 beschreibt kosmetische und/oder dermatologische Zubereitungen in Form von transparenten Gelen, die unter anderem mindestens ein Polyol und mindestens einen CS-7-Kohlenhydrat-Fettsäureester enthalten.

Weiterer Stand der Technik findet sich in den Schriften WO98/14167, EP 676194, WO 98/22075.

Aus dem vorliegenden Schrifttum geht hervor, daß es nicht an Versuchen gefehlt hat, die genannten lipophilen Wirkstoffe stabil in kosmetische Formulierungen einzuarbeiten, dies aber bisher nicht in befriedigender Weise gelang. Ausgehend hiervon zeigt die Erfindung einen Weg auf, derartige Wirkstoffe stabil, einfach und bioverfügbar in O/W-Emulsionen einzuarbeiten.

Es hat sich nämlich für den Fachmann völlig unvorhersehbar herausgestellt, daß O/W-Emulsionen, die als Emulgator Mischungen aus
(a) einer oder mehreren grenzflächenaktiven Substanzen A, gewählt aus der Gruppe der Glucosederivate, welche sich durch die Strukturformel 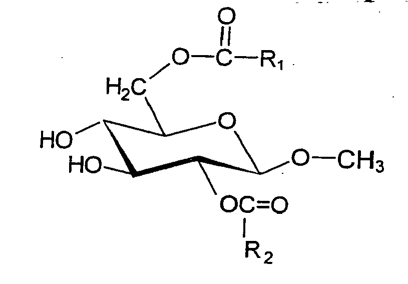 auszeichnen, wobei R₁ und R₂ unabhängibg voneinander entweder ein Wasserstoffatom oder einen verzweigten oder unverzweigten Alkylrest mit 1 bis 24 Kohlenstoffatomen darstellen,
(b) einer oder mehreren grenzflächenaktiven Substanzen B, gewählt aus der Gruppe der Substanzen der allgemeinen Strukturformel 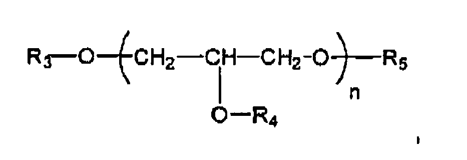 wobei R₃, R₄ und R₅ voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt, sowie
(c) einen lipophilen und/oder ungesättigten kosmetischen Wirkstoff mit einem log P-Wert größer als 3,5 enthalten, wobei dieser Retinol ist,
den Nachteilen des Standes der Technik abhelfen.

Weiterhin ist es bevorzugt, als grenzflächenaktive Substanzen A Methylglucosedistearat und als grenzflächenaktive Substanzen B Triglyceryldicarboxylate mit der generischen Formel 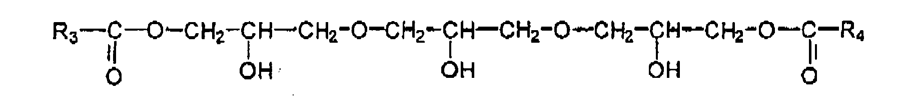 bei welcher R₃ und R₄ unabhängig voneinander gewählt werden aus der Gruppe, welche umfaßt: H, verzweigte bzw. unverzweigte, gesättigte bzw. ungesättigte Fettsäurereste mit 14 bis 20 Kohlenstoffatomen zu wählen.

Bevorzugt ist es, als Emulgator etwa äquimolare Mischungen aus Methylglucosedistearat und Triglyceryldicarboxylaten mit der generischen Formel 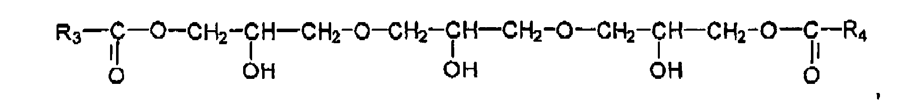 bei denen die Reste R₃ und R₄ vorzugsweise beide einen Stearatrest bezeichnen, zu verwenden, "Polyglyceryl(3)-Methylglucosedistearat" (PGMS) unter der Warenbezeichnung Tego Care® 450 von der Gesellschaft Th. Goldschmidt KG erhältlich.

Bevorzugt ist es, wenn der Gehalt an lipophilen und/oder ungesättigten Wirkstoffen von 0,001 - 5,0 Gew.% bezogen auf Gesamtgewicht der Zubereitung, beträgt.

Erfindungsgemäß sind auch kosmetische und/oder dermatologische Zubereitungen, die die oben genannten Emulsionen enthalten, wobei bevorzugt als Antioxidantien Tocopherol, Butylhydroxytoluol und/oder α-Glycosylrutin enthalten sein können.

Vorteilhaft werden diese Zubereitungen zur nicht therapeutischen Behandlung und/oder Vorbeugung von UV-Licht bedingten Hautschäden sowie von Hautalterungserscheinungen verwendet.

Es war für den Fachmann daher nicht vorauszusehen gewesen, daß die erfindungsgemäß verwendeten Emulsionen bzw. kosmetische oder dermatologische Zubereitungen, solche enthaltend
- besser gegen die Hautalterung wirken
- besser als Radikalfänger wirken
- besser die Bindung von schädlichen Photoprodukten an Lipide, DNS und Proteine verhindern
- besser die Zellerneuerung und Regeneration der Haut steigern
- besser Pigmentierungsstörungen, trockenen Hautzuständen und Hornschichtbarrierestörungen vorbeugt
- besser vor Falten, Altersflecken, Teleangiektasien schützen
- besser die Haut gegen Photoreaktionen und lichtbedingte Hautschäden schützen
als die Zubereitungen des Standes der Technik. Ferner war nicht vorauszusehen gewesen, daß die erfindungsgemäß verwendeten Emulsionen in kosmetischen oder dermatologischen Zubereitungen eine höhere Stabilität der lipophilen, ungesättigten Wirkstoffe aufweist als herkömmliche Emulsionen, was insbesondere die Retinolderivate und ganz besonders das Retinol betrifft.

Als besonders vorteilhafte Ausführungsform der vorliegenden Erfindung wird angesehen die Verwendung der beschriebenen Emulsionen zur Bekämpfung und/oder Prophylaxe des oxidativen Stresses sowie lichtbedingter Hautalterungseffekte, insbesondere von Hautfältchen.

Die erfindungsgemäßen kosmetischen oder dermatologischen Zubereitungen können wie üblich zusammengesetzt sein und zur Behandlung, der Pflege und der Reinigung der Haut und/oder der Haare und als Schminkprodukt in der dekorativen Kosmetik dienen. Sie enthalten bevorzugt 0,001 Gew.-% bis 10 Gew.-%, bevorzugt 0,01 Gew.-% bis 5 Gew.-%, insbesondere 0,02 - 2,0 Gew.-%, bezogen auf das Gesamtgewicht der Zubereitungen, an erfindungsgemäß verwendeten Wirkstoffkombinationen.

Es ist erfindungsgemäß bevorzugt, den beschriebenen Emulsionen Komplexbildner zuzufügen.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. - Ion zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen).

Der oder die Komplexbildner sind erfindungsgemäß vorteilhaft in kosmetischen oder dermatologischen Zubereitungen bevorzugt zu 0,01 Gew.-% bis 10 Gew.-%, bevorzugt zu 0,05 Gew.-% bis 5 Gew.-%, insbesondere bevorzugt zu 0,1 - 1,0 Gew.-%, bezogen auf das Gesamtgewicht der Zubereitungen, enthalten.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder eine multiple Emulsionen, beispielsweise vom Typ Wasser-in-Öl-in-Wasser (W/O/W), ein Gel, einen festen Stift oder eine Salbe darstellen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Erfindungsgemäße Emulsionen im Sinne der vorliegenden Erfindung sind z.B. Cremes, Lotionen, kosmetische Milchzubereitungen, Mousse-Creme, die aus einem Aerosolbehälter austragbar sind. Diese enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Insbesondere können erfindungsgemäß verwendete Emulsionen auch mit anderen Antioxidantien und/oder Radikalfängern kombiniert werden.

Vorteilhaft werden solche Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Carnosin, L-Carnosin und deren Derivate (z.B. Anserin), Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, y-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. y -Linolensäure, Linolsäure, Ölsäure), Folsäure und deren Derivate, Tocopherole und Derivate (z.B. Vitamin-E-acetat), sowie Koniferylbenzoat des Benzoeharzes, Rutinsäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Sesamol, Sesamolin, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,01 - 20 Gew.-%, insbesondere 0,02 - 5 Gew.-%, bezogen auf das Gesamtgewicht der Zubereitung.

Sofern Vitamin E und/oder dessen Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die zusätzlichen Antioxidantien darstellen, ist vorteilhaft, deren jeweilige Konzentrationen aus dem Bereich von 0,001 - 5 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl; '
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und lsotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Ganz besonders bevorzugte Ölkomponenten sind Octyldodecanol, Capryli/Capric-Triglycerid, Cyclometicone, C12-15 Alkylbenzoat und Dicaprylylcarbonat.

Erfindungsgemäße als Emulsionen vorliegenden Zubereitungen können zusätzlich zu dem Alkylglykosid einen oder mehrere weitere Emulgatoren enthalten. Diese Emulgatoren können vorteilhaft gewählt werden aus der Gruppe der nichtionischen, anionischen, kationischen oder amphoteren Emulgatoren.

Unter den nichtionischen Emulgatoren befinden sich
a) Partialfettsäureester und Fettsäureester mehrwertiger Alkohole und deren ethoxylierte Derivate (z. B. Glycerylmonostearate, Sorbitanstearate, Glycerylstearylcitrate, Sucrosestearate)
b) ethoxylierte Fettalkohole und Fettsäuren
c) ethoxilierte Fettamine, Fettsäureamide, Fettsäurealkanolamide
d) Alkylphenolpolyglycolether (z.B. Triton X)
e) Zuckerderivate (Ester und/oder Ether von Glucose, Saccharose und anderen Zuckern)
Unter den anionischen Emulgatoren befinden sich
a) Seifen (z. B. Natriumstearat)
b) Fettalkoholsulfate
c) Mono-, Di- und Trialkylphosphosäureester und deren Ethoxylate

Unter den kationischen Emulgatoren befinden sich
a) quaternäre Ammoniumverbindungen mit einem langkettigen aliphatischen Rest z.B. Distearyldimonium Chloride

Unter den amphoteren Emulgatoren befinden sich
a) Alkylamininoalkancarbonsäuren
b) Betaine, Sulfobetaine
c) Imidazolinderivate

Weiterhin gibt es natürlich vorkommende Emulgatoren, zu denen Bienenwachs, Wollwachs, Lecithin und Sterole gehören.

O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel

   R-COO-(-CH₂-CH₂-O-)ₙ -H,
- der veretherten Fettsäureethoxylate der allgemeinen Formel

   R-COO-(-CH₂-CH₂-O-)ₙ -R',
- der veresterten Fettsäureethoxylate der allgemeinen Formel

   R-COO-(-CH₂-CH₂-O-)ₙ -C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

   R-O-(-CH₂-CH₂-O-)ₙ-CH₂-COOH nd n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-S0₃-H
- der Fettalkoholpropoxylate der allgemeinen Formel

   R-O-(-CH₂-CH(CH₃)-O-)ₙ-H;
- der Polypropylenglycolether der allgemeinen Formel

   R-O-(-CH₂-CH(CH₃)-O-)ₙ-R',
- der propoxylierten Wollwachsalkohole,
- der veretherten Fettsäurepropoxylate

   R-COO-(-CH₂-CH(CH₃)-O-)ₙ-R',
- der veresterten Fettsäurepropoxylate der allgemeinen Formel

   R-COO-(-CH₂-CH(CH₃)-O-)ₙ-C(O)-R',
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpröpoxylate
- der propoxylierten Triglyceride
- der Alkylethercarbonsäuren der allgemeinen Formel

   R-O-(-CH₂-CH(CH₃)O-)ₙ-CH₂-COOH
- der Alkylethersulfate bzw. die diesen Sulfaten zugrundeliegenden Säuren der allgemeinen Formel R-O-(-CH₂-CH(CH₃)-O-)ₙSO₃-H
- der Fettalkoholethoxylate/propoxylate der allgemeinen Formel

   R-O-Xₙ-Yₘ-H,
- der Polypropylenglycolether der allgemeinen Formel

   R-O-Xₙ-Yₘ- R',
- der veretherten Fettsäurepropoxylate der allgemeinen Formel

   R-COO-Xₙ-Yₘ-R',
- der Fettsäureethoxylate/propoxylate der allgemeinen Formel

   R-COO-Xₙ-Yₘ-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)-stearylether (Steareth-20),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol-(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol-(20)isostearylether (Isosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (Isoceteth-13), Polyethylenglycol(14)isocetylether (isoceteth-14), Polyethylenglycol(15)isocetylether (lsoceteth-15), Polyethylenglycol(16)isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Pofyethylenglycol(19)cetylstearylether (Ceteareth-19), Polyethylenglycol(20)cetylstearylether (Ceteareth-20).

Es ist ferner von Vorteil, die Fettsäureethoxylate aus folgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,
Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol-(17)isostearat, Pofyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol-(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, ETD 2001, ETD 2020, ETD 2050, Ultrez 10 jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, lsobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicyisäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, - 2,4, 6-Trianilino-(p-carbo-2'-ethyl-1 '-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet.

Die Liste der genannten UVB-Filter, die in Kombination mit den erfindungsgemäßen Wirkstoffkombinationen verwendet werden können, soll selbstverständlich nicht limitierend sein.

Gegenstand der Erfindung ist auch die Verwendung einer Kombination der erfindungsgemäß verwendeten Emulsinen mit mindestens einem UVB-Filter als Antioxidans bzw. die Verwendung einer Kombination der erfindungsgemäß verwendeten Wirkstoffkombinationen mit mindestens einem UVB-Filter als Antioxidans in einer kosmetischen oder dermatologischen Zubereitung.
Es kann auch von Vorteil sein, UVA-Filter einzusetzen, die üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)-propan-1,3-dion und um 1-Phenyl-3-(4`-isopropylphenyl)propan-1,3-dion.

Weiterhin vorteilhafte UVA-Filter entstammen der Gruppe der Triazine, so z.B. das 2,4-Bis-{[4-(2-Ethyt-hexytoxy)-2-hydroxy]-phenyl}-6-(4-methoxyphenyt)-1,3,5-triazin (Handelsbezeichnung Tinosorb® S) sowie der Gruppe der Triazole, wie z.B. das 2,2'-Methylen-bis- [6-2H-benzotriazol-2yl]-4-(1,1,3,3-tetramethylbutyl)phenol) (Handelsbezeichnung Tinosorb® M). Ein vorteilhafter wasserlöslicher UVA-Filter stellt das 2'-bis-(1,4-Phenylen)-1 H-benzimidazol-4,6-disulfonsäure-Natriumsalz dar (Handelsbezeichnung Neo Heliopan AP®).

Es können die für die UVB-Kombination verwendeten Mengen eingesetzt werden.

Gegenstand der Erfindung ist auch die Verwendung einer Kombination von erfindungsgemäß verwendeten Emulsionen mit mindestens einem UVA-Filter als Antioxidans bzw. die Verwendung einer Kombination der erfindungsgemäßen Wirkstoffkombinationen mit mindestens einem UVA-Filter als Antioxidans in einer kosmetischen oder dermatologischen Zubereitung.

Gegenstand der Erfindung ist auch die Verwendung einer Kombination aus erfindungsgemäßen Emulsionen mit mindestens einem UVA-Filter und/oder mindestens einem UVB-Filter als Antioxidans in einer kosmetischen oder dermatologischen Zubereitung.

Erfindungsgemäße Emulsionen können auch anorganische Pigmente enthalten, die üblicherweise in der Kosmetik zum Schutze der Haut vor UV-Strahlen verwendet werden. Dabei handelt es sich um Oxide des Titans, Zinks, Zirkoniums, Siliciums, Mangans, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UVA-Filter und Pigment bzw. Zubereitungen, die diese Kombination enthalten, sind Gegenstand der Erfindung. Es können die für die vorstehenden Kombinationen genannten Mengen verwendet werden.

Bei kosmetischen und dermatologischen Zubereitungen zum Schutze der Haare vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die erfindungsgemäßen Emulsionen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide,Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Hautreinigungsmittel oder Shampoonierungsmittel darstellen, enthalten vorzugsweise mindestens eine anionische, nicht-ionische oder amphotere oberflächenaktive Substanz, oder auch Gemische aus solchen Substanzen, die erfindungsgemäß verwendeten Wirkstoffkombinationen im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Diese kosmetischen oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nichtionisches oder amphoteres Polymer oder auch Gemische derselben, sowie erfindungsgemäß verwendete Wirkstoffkombinationen in wirksamer Konzentration. Die Menge der verwendeten Polymeren liegt z. B. zwischen 0,1 und 10 Gew.-%, bevorzugt zwischen 0,1 und 3 Gew.-%.

Kosmetische Zubereitungen zur Behandlung und Pflege der Haare, die die erfindungsgemäß verwendeten Wirkstoffkombinationen enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Erfindungsgemäß können kosmetische Zubereitungen zur Behandlung und Pflege der Haare als Gele vorliegen, die neben einem wirksamen Gehalt an Isoquercitrin und dafür üblicherweise verwendeten Lösungsmitteln, bevorzugt Wasser, noch organische Verdickungsmittel, z.B. Gummiarabikum, Xanthangummi, Natriumalginat, CelluloseDerivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die vorliegende Erfindung umfaßt auch ein kosmetisches Verfahren zum Schutze der Haut und der Haare vor oxidativen bzw. photooxidativen Prozessen, das dadurch gekennzeichnet ist, daß man ein kosmetisches Mittel, welches erfindungsgemäße Emulsionen enthält, in ausreichender Menge auf die Haut oder Haare aufbringt.
Obwohl sich die erfindungsgemäß erzielten kosmetischen oder dermatologischen Zubereitungen und die darin befindlichen Wirkstoffkombinationen durch erhöhte Stabilität gegenüber oxidativem Einfluß auszeichnen, sind doch Lagerungsformen bevorzugt, in welchen ein verminderter Zutritt von Luftsauerstoff gegeben ist. So ist beispielsweise die Befüllung unter Inertgas, insbesondere Stickstoff, vorteilhaft. Als vorteilhafte Verpackung haben sich insbesondere Aluminiumtuben herausgestellt.

Es ist dem Fachmanne natürlich bekannt, daß anspruchsvolle kosmetische Zubereitungen zumeist nicht ohne die üblichen Hilfs- und Zusatzstoffe denkbar sind. Darunter zählen beispielsweise Konsistenzgeber, Füllstoffe, Parfum, zusätzliche Wirkstoffe wie Vitamine oder Proteine, Insektenrepellentien, Alkohol, Wasser, Salze, antimikrobiell, proteolytisch oder keratolytisch wirksame Substanzen usw.

### Beispiele

Alle Mengenangaben, Prozentangaben oder Teile beziehen sich, soweit nicht anders angegeben, auf das Gewicht, insbesondere auf das Gesamtgewicht der Zubereitungen oder der jeweiligen Mischungen."

Die nachfolgenden Beispiele sollen die Erfindung erläutern, ohne sie einzuschränken.

**Beispiele 1-2: O/W-Emulsionen**

| **Beispiel Nummer** | **1** |
|---|---|
| Polyglyceryl-3-Methylglucosedistearat | 2 |
| Stearylalcohol | |
| Cetearylalkohol | 5 |
| Cetylalcohol | |
| Myristylmyristat | 1 |
| C12-15 Alkylbenzoat | 2 |
| Caprylic/Capric Triglyceride | |
| Ethylhexylkokosfettsäureester | 4 |
| Octyldodecanol | |
| Vaseline | |
| Octamethyltetrasiloxan (Cyclomethicon) | 3 |
| Dicaprylylcarbonat | |
| Polydecen | 2 |
| TiO₂ | 1 |
| Ethylhexylmethoxycinnamat | 2 |
| 2-Ethylhexyl-2-cyano-3-diphenylacrylat (Octocrylen) | |
| Ethylhexyltriazon | 2 |
| Butylmethoxydibenzoylmethan | 1 |
| Bis-Ethylhexyloxypheno)-methoxyphenyltriazine | |
| Retinol | 0,1 |
| β-Carotin | |
| Ubichinon (Q10) | |
| Tocophorylacetat | 0,5 |
| α-Glucosylrutin | |
| Trinatrium EDTA | |
| Iminodisuccinat | 0,2 |
| Phenoxyethanol | |
| p-Hydroxybenzoesäutealkylester (Paraben) | 0,4 |
| Hexamidindüsethianat | |
| 1,3-ID!methylol-5,5-dimethyl-hydantoin(DMOM Hydantoin) | 0,2 |
| Diazolldinylharnstoff | |
| Ethano) dertaturiert | 2 |
| Xanthan Gummi | 0,1 |
| (carbomer) | 0,3 |
| Polyacrylamid | |
| Glycerin | 10 |
| Panthenol | 1 |
| Natürliche Öle, Pflanzenextrakte (Hamamelis, Sonnenblumenöl) | 0,1 |
| Füllstoffe/Additive (Distärkephosphat, SiO₂, Talkum, Butylhydroxytoluol) | 0,05 |
| Parfüm | q.s. |
| Wasser | Ad 100 |

| **Beispiel Nummer** | **2** |
|---|---|
| Polyglyceryl-3-Methylglucosedistearat | 2 |
| Sorbitanstearat | 1 |
| Cetearylalkohol | |
| Cetylalcohol | 3 |
| C12-15 Alkylbenzoat | 2 |
| Caprylic/Capric Triglyceride | |
| Ethylhexylkokosfettsäureester | 3 |
| Vaseline | 2 |
| Octamethyltetrasiloxan (Cyclomethicon) | 3 |
| Polydecen | 1 |
| Dicaprylylcarbonat. | |
| TiO₂ | |
| Ethylhexylmethoxycinnamat | 4 |
| 2-Ethylhexyl-2-cyano-3-diphenylacrylat (Octocrylen) | |
| Ethylhexyltriazon | |
| Phenylbenzimidazolsulfonsäure | |
| Butylmethoxydibenzoylmethan | |
| Bis-Ethylhexyloxyphenol-methoxyphenyltriazlne | 1 |
| Ubichinon (Q10) | |
| Tocopherol | 0,1 |
| Retinol | 0,05 |
| β-Carotin | |
| Trinatrium EDTA | 0,2 |
| Iminodisuccinat | |
| Phenoxyethanol | 0,3 |
| p-Hydroxybenzoesäurealkylester (Paraben) | 05 |
| Hexamidindiisethionat | 0,2 |
| 1,3.Dimethylols-5,dimethyil-hydantoin(DMDM Hydantoin) | |
| Diazolidinylhamstoff | |
| Ethanol denafuriart | |
| Xanthan Gummi | |
| Pölyacrylsäure (Carbomer) | 0,3 |
| Glycerin | 8 |
| Panthenol | |
| Natürliche Öle, Pflanzen-extrakte (Hamamelis, Sonnenblumenöl) | |
| Füllstoff4/Additive (Distärkephosphat, SiO₂, Talkum, Butylhydroxytoluol) | |
| Parfüm | q.s. |
| Wasser | Ad 100 |

## Patentansprüche

1. O/W-Emulsionen, die als Emulgator Mischungen aus
(a) einer oder mehreren grenzflächenaktiven Substanzen A, gewählt aus der Gruppe der Glucosederivate, welche sich durch die Strukturformel 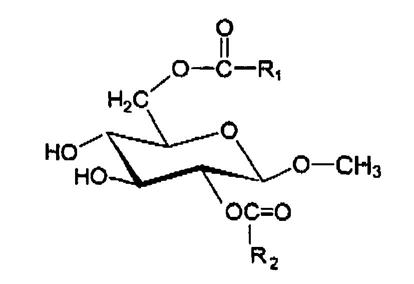 auszeichnen, wobei R₁ und R₂ unabhängig voneinander entweder ein Wasserstoffatom oder einen verzweigten oder unverzweigten Alkylrest mit 1 bis 24 Kohlenstoffatomen darstellen,
(b) einer oder mehreren grenzflächenaktiven Substanzen B, gewählt aus der Gruppe der Substanzen der allgemeinen Strukturformel 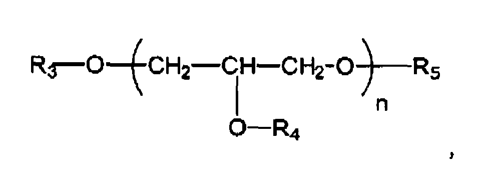 wobei R₃, R₄ und R₅ voneinander gewählt werden aus der Gruppe, welche umfasst: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt, sowie
(c) einen lipophilen, ungesättigten, kosmetischen Wirkstoff mit einem log P-Wert größer als 3,5 enthalten wobei dieser Retinol ist.

2. Emulsionen nach mindestens einem der vorangehenden Ansprüche, **dadurch gekennzeichnet, daß**
als grenzflächenaktive Substanz A Methylglucosedistearat und
als grenzflächenaktive Substanzen B Triglyceryldicarboxylate mit der generischen Formel 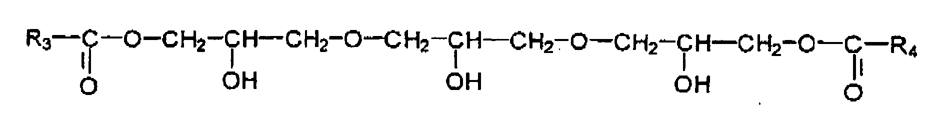 bei welcher R₃ und R₄ unabhängig voneinander gewählt werden aus der Gruppe, welche umfaßt: H, verzweigte bzw. unverzweigte, gesättigte bzw. ungesättigte Fettsäurereste mit 14 bis 20 Kohlenstoffatomen gewählt werden.

3. Emulsionen nach mindestens einem der vorangehenden Ansprüche, **dadurch gekennzeichnet, daß** sie als Emulgator etwa äquimolare Mischungen aus Methylglucosedistearat und Triglyceryldicarboxylaten mit der generischen Formel 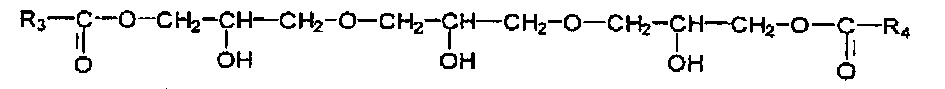 bei denen die Reste R₃ und R₄ vorzugsweise beide einen Stearatrest bezeichnen, enthalten.

4. Emulsionen nach mindestens einem der vorangehenden Ansprüche mit einem Gehalt an lipophilen, ungesättigten Wirkstoffen von 0.001 - .. 2,0 Gew.% bezogen auf Gesamtgewicht der Zubereitung.

5. Kosmetische und/oder dermatologische Zubereitungen, die Emulsionen nach mindestens einem der vorangehenden Anspruche enthalten.

6. Kosmetische und/oder dermatologische Zubereitung nach Anspruch 7 **dadurch gekennzeichnet, daß** sie als Antioxidantien Tocopherol, Butylhydroxytoluol und/oder α-Giycosylrutin enthalten.

7. Nicht therapeutische Verwendung von Zubereitungen nach mindestens einem der vorangehenden Ansprüche zur Behandlung und/oder Vorbeugung von UV-Licht bedingten Hautschäden sowie von Hautalterungserscheinungen.

8. Nicht therapeutische Verwendung von Zubereitungen nach mindestens einem der vorangehenden Ansprüche zur Reduktion und/oder Vorbeugung der Bildung von Hautfältchen.

## Claims

1. O/W emulsions which comprise, as emulsifier, mixtures of
(a) one or more interface-active substances A selected from the group of glucose derivatives **characterized by** the structural formula 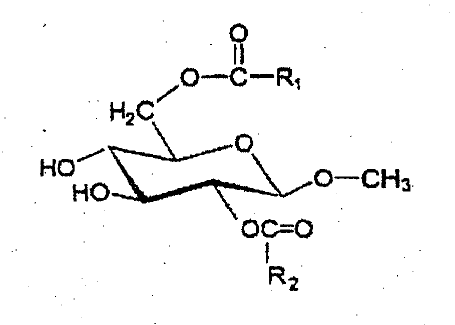 where R₁ and R₂, independently of one another, are either a hydrogen atom or a branched or unbranched alkyl radical having 1 to 24 carbon atoms,
(b) one or more interface-active substances B selected from the group of substances of the general structural formula 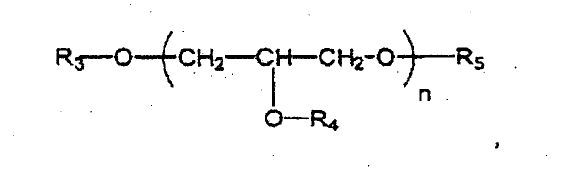 where R₃, R₄ and R₅, independently of one another, are selected from the group which comprises: H, branched or unbranched, saturated or unsaturated fatty acid radicals having 8 to 24 carbon atoms in which up to three aliphatic hydrogen atoms can be substituted by hydroxy groups and n is a number from 2 to 8, and '
(c) a lipophilic, unsaturated cosmetic active ingredient with a log P value greater than 3.5, where this is retinol.

2. Emulsions according to at least one of the preceding claims, **characterized in that**
as interface-active substance A, methylglucose distearate is chosen and
as interface-active substances B, triglycerol dicarboxylates are chosen with the generic formula 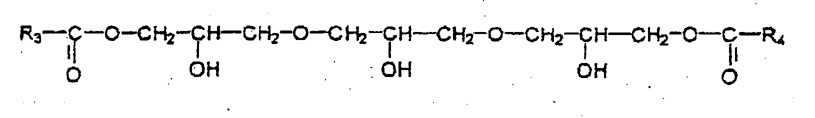 in which R₃ and R₄, independently of one another, are selected from the group which comprises: H, branched or unbranched, saturated or unsaturated fatty acid radicals having 14 to 20 carbon atoms.

3. Emulsions according to at least one of the preceding claims, **characterized in that** they comprise, as emulsifier, approximately equimolar mixtures of methylglucose distearate and triglycerol dicarboxylates with the generic formula 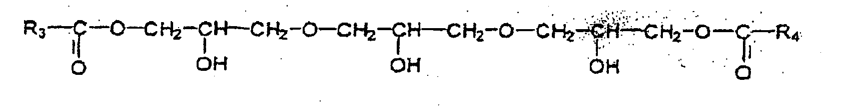 in which the radicals R₃ and R₄ preferably both designate a stearate radical.

4. Emulsions according to at least one of the preceding claims with a content of lipophilic unsaturated active ingredients of 0.001 - 2.0% by weight, based on the total weight of the preparation.

5. Cosmetic and/or dermatological preparations which comprise emulsions according to at least one of the preceding claims.

6. Cosmetic and/or dermatological preparations according to Claim 5, **characterized in that** they comprise, as antioxidants, tocopherol, butylhydroxytoluene and/or α-glycosylrutine.

7. Nontherapeutic use of preparations according to at least one of the preceding claims for the treatment and/or prevention of UV-light-induced skin damage and of skin ageing symptoms.

8. Nontherapeutic use of preparations according to at least one of the preceding claims for reducing and/or preventing the formation of skin wrinkles.

## Revendications

1. Emulsions huile dans l'eau comprenant, en tant qu'agent émulsifiant, des mélanges de :
(a) une ou plusieurs substances tensioactives A choisies parmi le groupe des dérivés du glucose qui sont **caractérisés par** la formule structurale 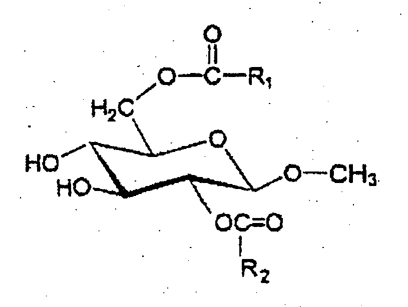 dans laquelle R₁ et R₂ représentent, indépendamment l'un de l'autre, soit un atome d'hydrogène, soit un radical alkyle ramifié ou non ramifié renfermant de 1 à 24 atomes de carbone,
(b) une ou plusieurs substances tensioactives B choisies parmi le groupe des substances de formule structurale générale 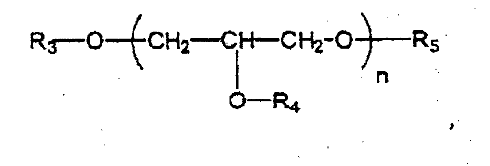 dans laquelle R₃, R₄ et R₅ sont choisis, indépendamment les uns des autres, parmi le groupe comprenant : un atome d'hydrogène, des radicaux acides gras ramifiés ou non ramifiés, saturés ou insaturés, renfermant de 8 à 24 atomes de carbone, dans lesquels jusqu'à trois atomes d'hydrogène aliphatiques peuvent être substitués par des groupes hydroxy et n représente un nombre de 2 à 8, ainsi que
(c) un ingrédient actif cosmétique insaturé lipophile présentant une valeur de log P supérieure à 3,5, celui-ci étant le rétinol.

2. Emulsions selon au moins l'une des revendications précédentes, **caractérisées en ce que** :
le distéarate de,méthylglucose est choisi en tant que substance tensioactive A, et '
des dicarboxylates de triglycéryle de formule générique 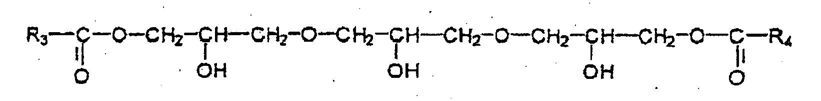
dans laquelle R₃ et R₄ sont choisis, indépendamment l'un de l'autre, parmi le groupe comprenant : un atome d'hydrogène, des radicaux acides gras ramifiés ou non ramifiés, saturés où insaturés, renfermant de 14 à 20 atomes de carbone, sont choisis en tant que substances tensioactives B.

3. Emulsions selon au moins l'une des revendications précédentes, **caractérisées en ce qu'**elles comprennent, en tant qu'agent émulsifiant, des mélanges approximativement équimolaires de distéarate de méthylglucose et de dicarboxylates de triglycéryle de formule générique 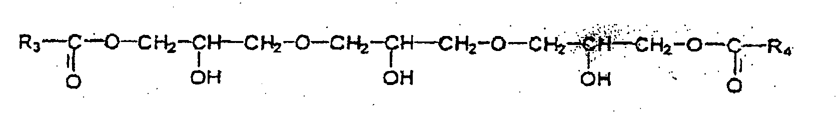 dans lesquels les radicaux R₃ et R₄ représentent, de préférence, tous les deux un radical stéarate.

4. Emulsions selon au moins l'une des revendications précédentes, présentant une teneur en ingrédients actifs insaturés lipophiles de 0,001 à 2,0 % en poids, par rapport au poids total de la préparation.

5. Préparations cosmétiques et/ou dermatologiques comprenant des émulsions selon au moins l'une des revendications précédentes.

6. Préparations cosmétiques et/ou dermatologiques selon la revendication 5, **caractérisées en ce qu'**elles comprennent, en tant qu'agents antioxydants, du tocophérol, du butylhydroxytoluène et/ou de la α-glycosylrutine.

7. Utilisation de préparations non thérapeutiques selon au moins l'une des revendications précédentes pour le traitement et/ou la prévention d'atteintes, cutanées causées par la lumière UV, ainsi que de symptômes de vieillissement de la peau.

8. Utilisation de préparations non thérapeutiques selon au moins l'une des revendications précédentes pour la réduction et/ou la prévention de la formation de rides cutanées.
